# EUROPEAN PATENT SPECIFICATION

(11) **EP 3 031 794 B1**
(45) Date of publication and mention of the grant of the patent: **02.01.2019**
(21) Application number: 14834562.2
(22) Date of filing: 06.08.2014
(51) Int. Cl.: C07C 217/14, A61K 31/138, A61P 11/06, A61P 11/08, A61P 29/00, C07C 217/20, C07C 217/80, C07C 233/25, C07C 217/18

(54) **DIPHENYLOXYALKYLAMINE DERIVATIVES AND ARYLOXYALKYLAMINE DERIVATIVES, PHARMACEUTICAL COMPOSITION, USE OF SAID PHARMACEUTICAL COMPOSITION FOR TREATING, PREVENTING OR INHIBITING CHRONIC PULMONARY INFLAMMATORY DISEASES AND METHOD FOR TREATING OR PREVENTING SUCH DISEASES**
DIPHENYLOXYALKYLAMINDERIVATE UND ARYLOXYALKYLAMINDERIVATE, PHARMAZEUTISCHE ZUSAMMENSETZUNG, VERWENDUNG DIESER PHARMAZEUTISCHEN ZUSAMMENSETZUNG ZUR BEHANDLUNG, VORBEUGUNG ODER HEMMUNG CHRONISCHER ENTZÜNDLICHER LUNGENERKRANKUNGEN UND VERFAHREN ZUR BEHANDLUNG ODER PRÄVENTION DERARTIGER ERKRANKUNGEN
DÉRIVÉS BIPHÉNYLOXY-ALKYL-AMINES ET ARYLOXY-ALKYL-AMINES, COMPOSITION PHARMACEUTIQUE, UTILISATION DE CETTE COMPOSITION PHARMACEUTIQUE DANS LE TRAITEMENT OU LA PRÉVENTION OU L'INHIBITION DE MALADIES INFLAMMATOIRES PULMONAIRES CHRONIQUES ET MÉTHODE DE TRAITEMENT OU DE PRÉVENTION DE CES MALADIES

(30) Priority: 09.08.2013 BR 102013020313
(43) Date of publication of application: 15.06.2016
(73) Proprietor: Fundação Oswaldo Cruz, 21045-900 Rio de Janeiro, RJ (BR)
(72) Inventor: MARTINS, Marco, Aurélio, 22245-110 Laranjeiras Rio de Janeiro RJ (BR); DA COSTA, Jorge, Carlos, Santos, 22210-080 Laranjeiras Rio de Janeiro RJ (BR); DA SILVA, Emerson, Teixeira, 20270-004 Tijuca Rio de Janeiro RJ (BR); FARIA, Robson, Xavier, 23520-317 Santa Cruz Rio de Janeiro RJ (BR); DE SOUZA, Marcus, Vinicius, Nora, 21932-819 Ilha do Governador Rio de Janeiro RJ (BR); SERRA, Magda, Fraguas, 20511-270 Tijuca Rio de Janeiro RJ (BR)
(74) Representative: Stuttard, Garry Philip
(86) International application number: PCT/BR2014/000266
(87) International publication number: WO 2015/017906

(56) References cited:
- EP-A1- 2 154 131
- WO-A1-98/33765
- WO-A1-2004/014841
- WO-A1-2006/046916
- WO-A1-2009/012998
- WO-A1-2013/151923
- WO-A2-2006/105304
- WO-A2-2012/050884
- US-A- 3 659 019
- US-A1- 2006 270 693
- US-B1- 8 012 901
- DATABASE CHEMCATS [Online] 28 March 2014 XP003032426 Retrieved from STN Database accession no. 1501635987
- DATABASE CHEMCATS [Online] 07 June 2014 XP003032427 Retrieved from STN Database accession no. 258491288
- DATABASE CHEMCATS [Online] 07 June 2014 XP003032428 Retrieved from STN Database accession no. 1090755658
- DATABASE CHEMCATS [Online] 28 March 2014 XP003032429 Retrieved from STN Database accession no. 1048679372
- DATABASE CHEMCATS [Online] 28 March 2014 XP003032430 Retrieved from STN Database accession no. 847252789
- DATABASE CHEMCATS [Online] 07 June 2014 XP003032431 Retrieved from STN Database accession no. 0704790203
- DATABASE CHEMCATS [Online] 28 March 2014 XP003032432 Retrieved from STN Database accession no. 0182802168
- DATABASE CHEMCATS [Online] 07 June 2014 XP003032433 Retrieved from STN Database accession no. 0688047258
- DATABASE CHEMCATS [Online] 28 March 2014 XP003032434 Retrieved from STN Database accession no. 1837129703
- DATABASE CHEMCATS [Online] 07 June 2014 XP003032435 Retrieved from STN Database accession no. 1652613476
- DATABASE CHEMCATS [Online] 28 March 2014 XP003032436 Retrieved from STN Database accession no. 1409443556
- DATABASE CHEMCATS [Online] 07 June 2014 XP003032437 Retrieved from STN Database accession no. 1015245112
- DATABASE CHEMCATS [Online] 07 June 2014 XP003032438 Retrieved from STN Database accession no. 0771954682
- DATABASE CHEMCATS [Online] 28 March 2014 XP003032439 Retrieved from STN Database accession no. 0327193429
- DATABASE CHEMCATS [Online] 28 March 2014 XP003032440 Retrieved from STN Database accession no. 0318828718

## Description

The present invention relates to certain diphenyloxyalkylamine derivatives and aryloxyalkylamine derivatives that are structurally analogous to mexiletine, with important biological activities. Such analogues showed higher relaxation potency of the respiratory smooth muscle and marked anti-inflammatory action in the lung tissue, compared to mexiletine prototype. Importantly, the derivatives of the present invention are devoid of undesirable side effects present in the prototype, as well as in other drugs of the same therapeutic class as the prototype.

The mexiletine, 2-(2-aminopropoxy)-1,3-dimethylbenzene, represented in formula (I) below, registered with the trade name Mexitil®, is a counterpart of the local anesthetic lidocaine clinically used by mouth for controlling cardiac arrhythmias (Campbell, 1987) and relieving pain of different origins, including neuropathic pain (Jarvis *et al.,* 1998) and cephalalgias difficult to treat (Marmura et *al.,* 2008).

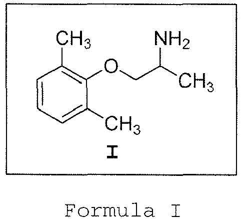

US-A-3659019 and US-A-3954872 describe the structure and use of mexiletine. Pharmaceutical formulations using mexiletine can be found in US-A-4031244 (for controlling arrhythmias) and WO-A-2012/050884 (for the treatment of inflammatory diseases such as asthma).

The mexiletine acts by inhibiting the propagation of action potential in Purkinje network with low interference on the autonomic nervous system, by blocking the fast sodium channels (Monk *et al*., 1990). Also orally, mexiletine is capable of inducing relaxation of airways in asthmatic patients, suggesting a potential, as the therapeutic use, in the pharmacological control of asthma (Groeben et *al.,* 1996). However, this application comes up against important limitations arising from the very action of mexiletine as inhibitor of sodium channel, which is inherently linked to serious side effects such as cardiovascular toxicity as well as gastrointestinal and central disorders (Campbell, 1987).

The lungs play a central role in gas exchange and make direct connection with the external environment Because of this, allergic, infectious and occupational disorders of the respiratory system are among the most frequent and disabling diseases that affect humans (Saraiva et al., 2011). Asthma is characterized by non-specific bronchial hyperreactivity and marked eosinophilic inflammatory infiltrate in the lungs. Recurrent episodes of breathlessness, wheezing and cough are the main symptoms of this disease, which if not treated can cause death (Lemanske et al., 2010; Mannam et al., 2010). Recent data indicate that the number of asthmatics is increasing in the world. The disease affects people of all ages and kills six people every day in Brazil (Brightling et *al.,* 2012). Chronic obstructive pulmonary disease (COPD) includes those patients who in general are affected simultaneously with emphysema and chronic bronchitis. Emphysema destroys the walls of the alveolar sacs, decreasing dramatically the surface area available for gas exchange. Bronchitis causes constriction of the pulmonary airways and blocks them with an exaggerated production of mucus (Brody, 2012). COPD is one of the biggest killers worldwide and is difficult to diagnose both in developed and developing nations (Dance, 2012) .

Thus, it is highly desirable to develop substances which act in the treatment, prevention or inhibition of pulmonary inflammatory disorders, without the disadvantages indicated by the state of the art.

The present invention relates to derivatives structurally analogous to mexiletine, that is, diphenyloxyalkylamines and aryloxyalkylamines, with anti-inflammatory and bronchodilator properties. For its lower activity on sodium channels, there are indications that new diphenyloxyalkylamine derivatives and aryloxyalkylamine derivatives are devoid of the side effects present in other drugs in the same therapeutic class. More specifically, the invention relates to structural modifications in the mexiletine molecule, producing new derivatives containing structural unprecedented pattern and low activity on the sodium channel. Interestingly, these analogues are capable of inhibiting the contraction of respiratory smooth muscle, in addition to blocking the pulmonary inflammatory response triggered by various stimuli, including allergens and cigarette smoke.

Thus in a first aspect the present invention provides diphenyloxyalkylamine derivatives and aryloxyalkylamine derivatives selected from the structures below:

| **Structure** | **Code** |
|---|---|
| 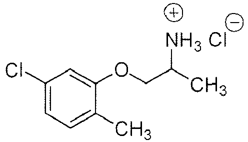 | JME-170 |
| C₁₀H₁₅Cl₂NO⁻ | |
| Mol. Wt.: 236,14 | |
| 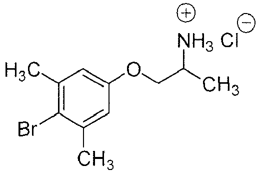 | JME-173 |
| C₁₁H₁₇BrClNO⁻ | |
| Mol. Wt.: 294,62 | |
| 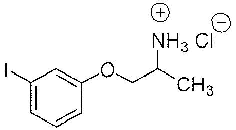 | JME-141 |
| C₉H₁₃ClINO⁻ | |
| Mol. Wt.: 313,56 | |
| 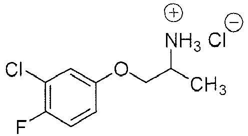 | JME-188 |
| C₉H₁₂Cl₂FNO⁻ | |
| Mol. Wt.: 240,10 | |
| 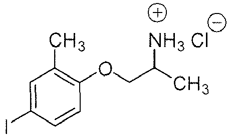 | JME-207 |
| C₁₀H₁₅ClINO⁻ | |
| Mol. Wt.: 327,59 | |
| 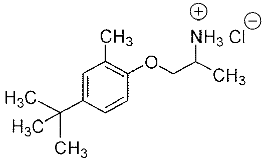 | JME-208 |
| C₁₄H₂₄ClNO⁻ | |
| Mol. Wt.: 257,80 | |
| 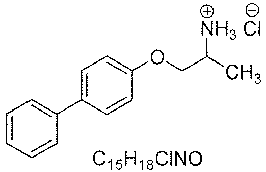 | JME-209 |
| Mol. WT.: 263,76 | |
| 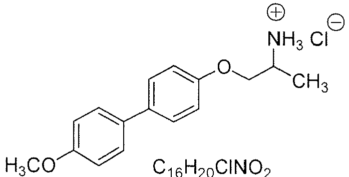 | JME-209-1 |
| Mol. Wt.: 293,79 | |
| 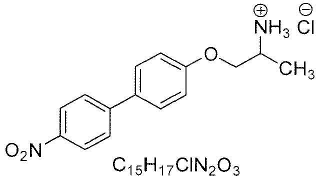 | JME-209-2 |
| Mol.Wt.: 308,76 | |
| 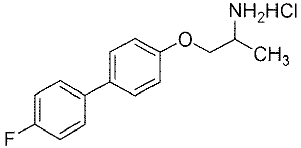 | JME-209-3 |
| C₁₅H₁₇ClFNO | |
| Mol. Wt.: 281,75 | |
| 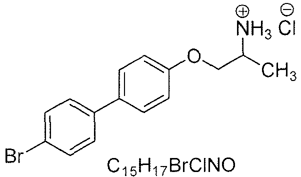 | JME-209-4 |
| Mol. Wt.: 342,66 | |
| 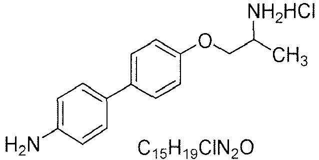 | JME-209-5 |
| Mol. Wt.: 278,78 | |
| 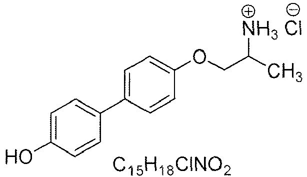 | JME-209-6 |
| Mol. Wt.: 279,76 | |
| 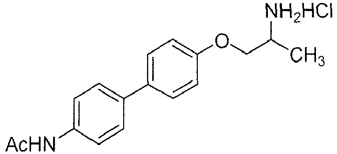 | JME-209-7 |
| C₁₇H₂₁ClN₂O₂ | |
| Mol. Wt.: 320,81 | |
| 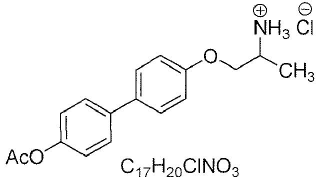 | JME-209-8 |
| Mol. Wt.: 321,8 | |

In a further aspect the present invention provides a pharmaceutical composition characterized by containing at least one of the derivatives as defined in the first aspect of the invention, or one of its salts formed by organic or mineral acids, and a pharmaceutically acceptable carrier.

In a yet further aspect the present invention provides composition as defined in the further aspect of the invention for use in the treatment, prevention or inhibition of pulmonary inflammatory disease by administration in a pharmacologically effective amount through any route of administration.
Table 1: Comparative effect of inhibition of sodium current evidenced by mexiletine and analogues, from the classes of diphenyloxyalkylamines and aryloxyalkylamines, in GH3 cells evaluated in the patch clamp system.
Table 2: Potency values (IC₅₀) and maximum effect (EMAX) of inhibition of the contraction response induced by carbachol (10 µM) on rat tracheal rings pretreated with mexiletine, JME-173 or JME-207, representatives of the class of aryloxyalkylamines. Data represent the mean ± SEM from 4 to 7 tracheal rings.
Table 3: Comparative values of inhibition potency (IC₅₀) and maximum effect (EMAX) of mexiletine, JME-207, JME-173 and JME-209, of the classes of diphenyloxyalkylamines and aryloxyalkylamines, relative to the blocking of anaphylactic mast cell degranulation.
Figure 1: Relaxing effect of the compounds aryloxyalkylamines JME-173 and JME-207, compared to that of mexiletine in trachea pre-contracted with carbachol.
Figure 2: Antispasmodic effect of aryloxyalkylamine JME-173 (Part A) and salmeterol (Part B) in the anaphylactic contraction of tracheal rings.
Figure 3: Mast cell stabilizing effect exhibited by compounds of the classes of diphenyloxyalkylamines and aryloxyalkylamines, JME-173, JME-207, JME-209, JME-141 and mexiletine.
Figure 4: Antispasmodic effect of diphenyloxyalkylamine JME-209 (30 and 100 mg/kg, orally) or carrier (0.9% NaCl), evaluated in mice subjected to methacholine challenge.
Figure 5: Antispasmodic effect of aryloxyalkylamine JME-207 (30 and 100 mg/kg, orally) or carrier (0.9% NaCl), evaluated in mice subjected to methacholine challenge.
Figure 6: Protocol for sensitization, antigen challenge and treatment used to assess the activity of the tested compounds in the experimental asthma model in mice.
Figure 7: Effect of nebulization of aryloxyalkylamines JME-141, JME-173, JME-188 or JME-207 on airway hyperresponsiveness in mice sensitized and challenged with ovalbumin.
Figure 8: Effect of nebulization of aryloxyalkylamines JME-141, JME-173, JME-188 or JME-207 on leukocyte infiltration evaluated in bronchoalveolar lavage in mice sensitized and challenged with ovalbumin.
Figure 9: Effect of nebulization of aryloxyalkylamines JME-141, JME-173, JME-188 or JME-207 on the generation of cytokines in lung tissue of mice sensitized and challenged with ovalbumin.
Figure 10: Effect of nebulization of aryloxyalkylamine JME-173 (0.5-2%) on airway hyperresponsiveness in mice sensitized and challenged with ovalbumin.
Figure 11: Effect of nebulization of aryloxyalkylamine JME-173 on the production of mucus in the airways in mice sensitized and challenged with ovalbumin.
Figure 12: Effect of nebulization of aryloxyalkylamine JME-173 on the sub-epithelial fibrosis airway response in mice sensitized and challenged with ovalbumin.
Figure 13: Effect of oral treatment with diphenyloxyalkylamine JME-209 on the increase of total count of leukocytes, mononuclear cells, eosinophils and neutrophils observed in bronchoalveolar lavage after stimulation with LPS in mice.
Figure 14: Effect of oral treatment with diphenyloxyalkylamine JME-209 on lung hyperresponsiveness observed after stimulation with LPS in mice.
Figure 15: Effect of oral treatment with diphenyloxyalkylamine JME-209 or dexamethasone on the increase of total count of leukocyte in bronchoalveolar lavage of mice exposed to cigarette smoke.
Figure 16: Effect of oral treatment with diphenyloxyalkylamine JME-209 or dexamethasone on the increase of total count of mononuclear cells, neutrophils and eosinophils in bronchoalveolar lavage of mice exposed to cigarette smoke.

It has been observed by the inventors that suitable structural modifications in the mexiletine molecule result in obtaining analogues with anti-inflammatory and bronchodilator properties. An important aspect is that such derivatives have low activity on the sodium channel, unlike the prototype, as seen in electrophysiological assays using the "patch clamp" technique in GH3 cells. Based on these data, the present invention proposes a new therapeutic method for the treatment of diseases related to obstruction and inflammation of airways, such as asthma and COPD, by topical or systemic administration of diphenyloxyalkylamine derivatives and aryloxyalkylamine derivatives, devoid of local anesthetic and antiarrhythmic activity, such as diphenyloxyalkylamine derivatives and aryloxyalkylamine derivatives of this invention.

The examples shown herein are intended only to exemplify, but without limiting the scope of the invention.

As used herein, the term alkyl means an alkyl group of linear, branched or cyclic chain, of up to eight (8) carbon atoms. Examples of alkyl groups used in the present invention are methyl, ethyl, propyl, butyl, "Alkyl ether", i.e. alkoxy can be interpreted herein as alkyl group, e.g., methoxy, ethoxy.

As used herein, the term alkene means an alkene group of linear, branched or cyclic chain, of up to eight (8) carbon atoms. Examples of alkene groups used in the present invention are methylene, ethylene, propylene.

As used herein, the term cyclic alkyl means a cycle alkane, alkene or containing heteroatoms, e.g., oxygen or sulfur.

As used herein, "room temperature" includes a range of 20 to 35°C;

As used herein, the term electron remover grouping includes nitro, cyano, azide, carbonyl, carboxyl, amidine, halogen groups;

As used herein, the term electron donor grouping includes methoxyl, ethoxyl, hydroxyl, alkylamine, amine groups.

The compounds of the present invention are defined by the following structures in Table I:

**Table I**

| **Structure** | **Code** |
|---|---|
| 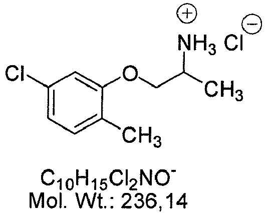 | JME-170 |
| 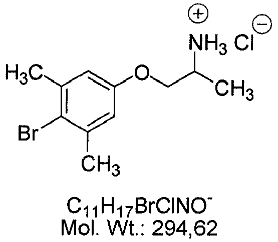 | JME-173 |
| 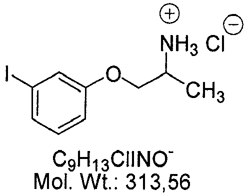 | JME-141 |
| 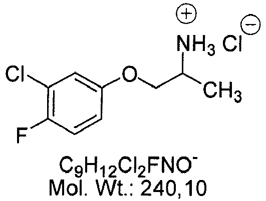 | JME-188 |
| 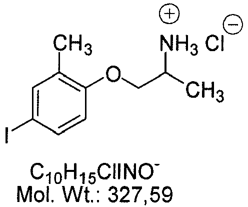 | JME-207 |
| 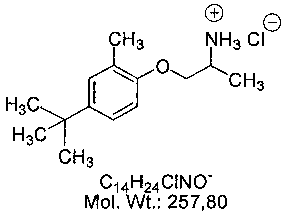 | JME-208 |
| 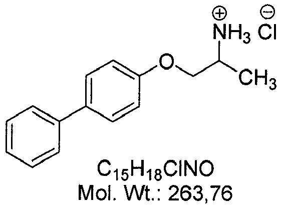 | JME-209 |
| 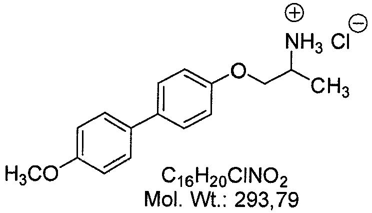 | JME-209-1 |
| 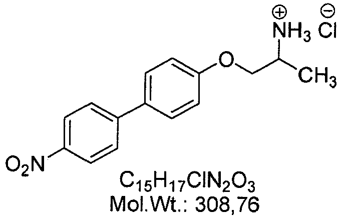 | JME-209-2 |
| 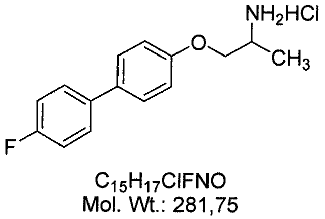 | JME-209-3 |
| 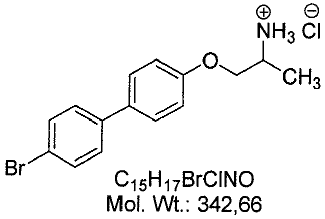 | JME-209-4 |
| 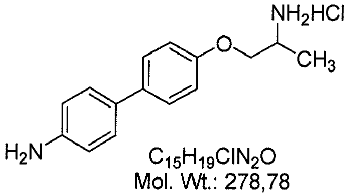 | JME-209-5 |
| 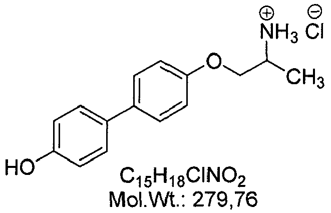 | JME-209-6 |
| 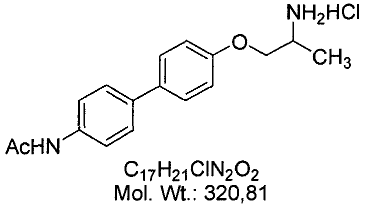 | JME-209-7 |
| 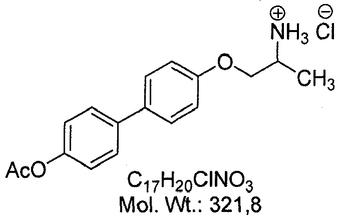 | JME-209-8 |

Examples contemplating the synthesis of the compounds structurally analogous to mexiletine, according to the present invention, will be shown below.

The examples shown herein are intended only to exemplify, but without limiting the scope of the invention.

### Example 1 - Synthesis of diphenyloxyalkylamine derivatives of structural formula II

The starting material substituted phenyl-phenol (29.38 mmol) was dissolved in acetone along with sodium carbonate (1-5 eq.) and catalytic amount of potassium iodide. After previous reflux, a solution of chloroacetone (1-3 eq.) was added over 0.5 to 2 hours, remaining under reflux for 2 to 5 hours. The medium was evaporated to dryness, to follow with the addition of water (30 mL) and extraction with ethyl acetate. The organic phase was dried and evaporated to give the first intermediate in the form of a dark oil (70-90%) .

The propanone, obtained as above (29.32 mmol), was dissolved in methanol and the medium cooled in ice bath, to follow with the addition of excess sodium borohydride (2-5 eq.). The reaction medium was stirred at room temperature for 2-5 hours. After addition of water, the reaction medium remained under stirring for another 30-60 minutes. The medium was concentrated at reduced pressure and extracted with ethyl acetate. A colorless oil was obtained after drying and evaporating the organic phase.

The above oil was dissolved in pyridine (10-30 mL) and the medium cooled in ice bath. Excess tosyl chloride (1 to 5 eq.) was added for up to 15 minutes. After 12-24 hours of reaction, the medium was added a solution of HCl until reaching pH 2 to 5. After stirring at room temperature, a white solid was precipitated in the reaction medium, which was removed by filtration. After drying, the solid was dissolved in methanol and reaction was performed with sodium azide (3 eq.) under reflux for 2-6 hours. After evaporation of the solvent, water was added and extracted with ethyl acetate. The organic phase was dried and evaporated to obtain the second intermediate, in the form of a yellowish oil (50- 70%).

The azide, obtained as above (16.6 mmol), was dissolved in methanol and catalyst Pd/C was added to this solution. The medium was bubbled with H₂ for up to 10 minutes and then allowed to stir in the presence of this gas for 2-10 hours. After filtering the palladium, the filtrate was evaporated to obtain an oil which was subsequently dissolved in acetone and filtered again. This solution was cooled in ice bath and treated with HCl gas flow until reaching pH 1-5. The precipitate was isolated by filtration followed by washing with cold acetone. The final product was obtained after drying, as a white solid, which spectral data are listed below:
**JME 209** [obtained from 4-phenyl-phenol according to the synthesis described in the Example 1]: M.P.: 252-254 °C; ¹H NMR (MeOD, 500 MHz) 7.1-7.6 (m, 9H, ArH), (4.0-4.2, m, 2H, -0- CH₂-), 3.75 (m, 1H, -CH-), 1.44 (d, 3H, CH₃)_{;} ¹³C NMR (MeOD, 125 MHz): 159.13, 141.97, 136.18, 130.60, 129.94, 129.34, 128.68, 128.38, 127.39, 127.10, 116.83, 115.55, 69.98, 47.87, 15.11; IR (KBr): 4368, 3047, 1924, 1608, 1049, 883, 812, 437; GC-MS (100%): m/z 227 (free base).
**JME 257** [obtained from 4-(4'-Bromo-phenyl)-phenol according to the synthesis described in the Example 1] M.P.:> 300 °C; ¹H NMR (MeOD, 400 MHz) 7.1-7.6 (m, 8H, ArH), 4.0-4.3 (m, 2H, -0-CH₂-), 3.75 (m, 1H, -CH-), 1.44 (d, 3H, CH₃, J=6.76Hz) ; ¹³C NMR (MeOD, 100 MHz): 159.45, 141.06, 134.86, 130.60, 133.07, 129.55, 129.26, 122.01, 116.36, 70.17, 48.56, 15.59; IR (KBr): 3000, 2989, 1603, 1485, 1247, 1041, 810, 734; GC-MS (99%): m/z 305 (free base).
JME 260 [obtained from 4-(4'-fluoro-phenyl)-phenol according to the synthesis described in the Example 1] M.P.: 225-227 °C; ¹H NMR (MeOD, 400 MHz): 7.0-7.6 (m, 8H, ArH), (4.0-4.3, m, 2H, -O-CH₂ -), 3.75 (m, 1H, -CH-), 1.45 (d, 3H, CH₃, J=6.80HZ) ; ¹³C NMR (MeOD, 100 MHz): 164.94, 162.51, 159.14, 138.31, 135.16, 129.54, 129.46, 129.25, 116.69, 116.48, 116.30, 70.16, 48.56, 15.61; IR (KBr): 2968, 2879, 1597, 1498, 1230, 1041, 815, 559, 513; GC-MS (100%): m/z 245 (free base).

### Example 2 - Synthesis of aryloxyalkylamine derivatives of structural formula III

The appropriately substituted phenol derivative (24.88 mmol) was dissolved in acetone along with potassium carbonate (1 to 5 eq.) and catalytic amount of potassium iodide. Under reflux, a solution of chloroacetone (1 to 3 eq.) was added in acetone for a period of 0.5-2 hours, remaining in this condition for a further period of 2-5 hours. Then, water was added and extracted with ethyl acetate. The organic phase was dried and evaporated to give the first intermediate in the form of a dark oil (70-95%).

The propanone, obtained as above (24.51 mmol), was dissolved in methanol and the medium cooled in ice bath, to follow with the addition of excess sodium borohydride (2-5 eq.). The reaction medium was stirred at room temperature for 2-5 hours. After addition of water, the reaction medium remained under stirring for another 30-60 minutes. The medium was concentrated at reduced pressure and extracted with ethyl acetate. A colorless oil was obtained after drying and evaporating the organic phase.

The above oil was dissolved in pyridine (20-50 mL) and the solution formed was cooled in ice bath. Excess tosyl chloride (1 to 5 eq.) was added for up to 15 minutes. After 12-24 hours of reaction, the medium was added a solution of HCl until reaching pH 2 to 5. After stirring at room temperature, a white solid was precipitated in the reaction medium, which was removed by filtration. After drying, the solid was dissolved in methanol and reaction was performed with sodium azide (3-7 eq.) under reflux for up to 20 hours. After evaporation of the solvent, water was added and extracted with ethyl acetate. The organic phase was dried and evaporated to obtain the second intermediate, in the form of a yellowish oil (50- 70%).

The azide, obtained as above (12.32 mmol), was dissolved in tetrahydrofuran and this solution was added triphenylphosphine (1-2 eq). The medium was then stirred at room temperature for up to 20 hours. Then, water was added and the reaction medium was heated until reflux, after which it was maintained for up to 3 hours. The organic solvent was removed by evaporation to form an oil which was dissolved again in acetone (30 mL). After cooling, the solution was subjected to HCl gas flow until pH 2-3, water was added and extracted with ethyl ether. The aqueous phase was basified until pH 10-12 and extracted with ethyl acetate. The organic phase was dried and concentrated to give an oil which was subsequently dissolved in acetone. The solution was cooled in ice bath and subjected to HCl gas flow until the medium pH is between 1 and 5, leading to the formation of a precipitate. The precipitate was isolated by filtration followed by washing with cold acetone. The final product was obtained after drying, as a white solid, which spectral data are listed below:
**JME 141** (obtained from 3-iodine-phenol according to the synthesis described in the example 2): M.P.: 204-206 °C; ¹H NMR (D₂O, 500 MHz): 7.0-7.5 (m, 4H, ArH), 4.0-4.3 (m, 2H, - O-CH2-), 3.8 (m, 1H, -CH-), 1.4 (d, 3H, -CH₃ J=6.5 Hz); ¹³C NMR (MeOD, 125 MHz): 158.17, 131.88, 130.58, 123.84, 114.39, 94.56, 73.06, 55.76, 16.28; IR (KBr): 3109, 2985, 1585, 1502, 1465, 1290, 1008, 763, 682; GC-MS (100%): m/z 277 (free base).
**JME 170** (obtained from 2-chloro-5-methyl-phenol according to the synthesis described in this example 2): M.P.:220-222 °C; ¹H NMR (D₂O, 400 MHz): 7.22 (m, 1H, ArH₆,), 7.05 (m, 2H, ArH_{2,4}) 4.0-4.3 (m, 2H, -O-CH₂-), 3.90 (m, 1H, -CH-), 2.27 (s, 3H, ArCH₃) , 1.51 (d, 3H, -CH₃, J=6.8Hz); ¹³C NMR (MeOD, 100 MHz): 156.32, 131.63, 131.33, 125.88, 121.31, 112.37, 68.98, 47.09, 30.26, 14.90, 14.39; IR (KBr): 3028, 1593, 1492, 1246, 1049, 854, 655; MS (ES): 200 (M + H)
**JME-173** (obtained from 3,5-dimethyl-4-bromo-phenol according to the synthesis described in the example 2) : M.P. :220-222 °C; ¹H NMR (D₂O, 500 MHz): 6.8 (m, 2H, ArH), 4.0-4.2 (m, 2H, -O-CH₂-), 3.80 (m, 1H, -CH-), 2.3 (s, 6H, ArCH₃), 1.4 (d, 3H, -CH₃ J=10Hz); ¹³C NMR (MeOD, 125 MHz): 156.28, 139.78, 118.54, 114.27, 68.52, 47.00, 23.06, 14.16; IR (KBr): 3066, 2978, 2120, 1739, 1585, 1468, 1319, 1172, 1018, 856, 812, 663; CG-MS (100%): m/z 227 (free base).
**JME 207** (obtained from 2-methyl-4-iodide-phenol according to the synthesis described in the example 2): M.P.:233-235 °C; ¹H NMR(MeOD, 500 MHz): 7.4 (m, 3H, ArH), 4.0-4.2 (m, 2H, -O-CH₂-), 3.7 (m, 1H, -CH-), 2.2 (s, 3H, ArCH₃), 1.4(d, 3H, -CH₃ J=6.8 Hz); ¹³C NMR (MeOD, 125 MHz): 157.59, 139.84, 136.44, 130.71, 114.40, 84.66, 47.79, 15.67 IR (KBr): 3066, 2978, 2120, 1739, 1585, 1468, 1319, 1172, 1018, 856, 812, 663; CG-MS (100%): m/z 291 (free base).

Melting points were determined in 130 fisatom apparatus and are uncorrected. Analyses of Proton Magnetic Resonance (1H NMR) were determined in Bruker AC 400 spectrometer at 400 MHz or 500 MHz. Multiplicities were designated as: s, singlet; d, doublet; t, triplet; dd, double doublet; m, multiplet; bs, broad signal. Analyses of Carbon Magnetic Resonance (13C NMR) were determined at 100 MHz or 125 MHz. Infrared spectra were obtained in a Perkin-Elmer 467 FTIR spectrometer using potash bromide pellets. Mass spectra were obtained in GC/MS column 122 5532 apparatus Agilent by electron impact. The progress of all reactions was monitored by thin layer chromatography, using aluminum chromate films (2.0 x 6.0 cm, 0.25 mm; silica gel 60, HF-254, Merck) with the aid of ultraviolet light at 264 nm. For purification by chromatography column, silica gel was used (230-400 mesh).

The following examples illustrate the pharmacological properties of the compounds of the present invention in comparison to prototype compound mexiletine. They also illustrate the potential of these analogues on the inhibition of pulmonary inflammatory diseases, such as asthma and COPD.

### Example 3 - Evaluation of the blocking potency of the sodium current exhibited by mexiletine compared with the analogues JME-141, JME-173, JME-188, JME-207, JME-209, JME-257 and JME-260.

### A. Method and Evaluation

Pituitary GH3 cells obtained from mice were grown in RPMI 1640 medium containing 10% fetal bovine serum, penicillin (100 U/ml) and streptomycin (100 µg/ml). The cells were kept at 37°C in a humidified atmosphere with 5% CO₂ and grown in slides for 1-2 before use. The ion channel currents in GH3 cells were recorded according to the "Patch Clamp" technique, as previously described (Neher et al., 1992). The slides containing adhered cells were placed in a chamber attached to a microscope, and continuously infused with saline with the following composition (mM): NaCl (150), KCl (5), MgCl₂ (1), CaCl₂ (0.01) EGTA (1), HEPES (10), BaCl₂ (2), and CdCl₂ (0.1). The solution pH was adjusted to 7.4 at room temperature with the aid of a NaOH solution. The cells were observed in inverted microscope in phase contrast mode (Axiovert 100, Carl Zeiss, Oberkochem, Germany). The voltage clamp records in the "whole cell" configuration with gigaohm sealing (> 10 GΩ) were obtained using an Axopatch-1D amplifier (Axon instruments, San Mateo, CA). Sodium currents were recorded in saline with or without the tested compounds. The series resistance was 6-10 MΩ for all experiments, when the pipette was filled with intracellular saline solution with the following composition (mM): KCl (150), NaCl (5), MgCl₂ (1), HEPES (10), and EGTA (0.1). The saline solution pH was adjusted to 7,4 at room temperature with the aid of a NaOH solution. Fifteen minutes after the rupture of the membrane patch, the records of the ionic currents were started. The pulse protocols and data acquisition were controlled by an interface (Axon Instruments, Palo Alto, CA) and acquired using Clampex 9 software. The records of sodium currents were filtered at 1 kHz and sampled at 8 kHz. Around 26% of series resistance was compensated electronically. The drugs were applied to the chamber by gravity. The infusion rate was maintained at 0.8 to 1.1 ml/minute and the bath volume was approximately 50 µl.

### B. Statistical Analysis

The results were expressed as mean ± Standard Error of the Mean. Statistical differences were determined by using tests of analysis of variance, followed by the Student-Newman-Keuls test. *p*-Values lower than or equal to 0.05 were considered significant.

### C. Results

Table 1 shows the concentrations of substances capable of inhibiting by 50% (IC₅₀) the sodium current in the target cells. By the patch clamp technique, it was found that the depolarization (-90 mV to 60 mV) of rat pituitary cells (GH3 cell line) generated sodium currents that were inhibited, on a concentration-dependent form by pre-treatment with tetrodotoxin (IC₅₀ = 304 nM) (data not shown), while the prototype substance mexiletine showed an IC₅₀ for blocking the sodium current of the order of 278 µM. Table 1 also shows that the analogous compounds showed IC₅₀ values between 178 and 1208 times higher than that shown by co-incubation with mexiletine.

**Table 1 - Comparative Effect of inhibition of sodium current evidenced by mexiletine and analogues of the invention, from the classes of diphenyloxyalkylamines and aryloxyalkylamines, in GH3 cells evaluated in the patch clamp system.**

| **Compounds** | **IC₅₀ (mM)** | **N** |
|---|---|---|
| Mexiletine | 0.278 | 4 |
| JME-141 | 222 | 3 |
| JME-207 | 177 | 4 |
| JME-173 | 183 | 4 |
| JME-188 | 199 | 4 |
| JME-209 | 49 | 4 |
| JME-257 | 336 | 4 |
| JME-260 | 107 | 4 |

Thus, the ranking of blocking potency of the sodium current in this system would be mexiletine >>> JME-209 > JME-260 > JME-207 > JME-173 > JME-188 > JME-141 > JME-257.

Since the main undesirable effects of mexiletine (including cardiovascular depression) result directly from the suppressing activity of sodium currents, it is possible to hypothesize that the analogous compounds have a lower toxicity potential compared to the prototype mexiletine. **Example 4** - Inhibitory activity of the contraction of tracheal smooth muscle of rat presented by the compounds mexiletine, JME-173 and JME-207.

### A. Method and Evaluation

All experimental procedures involving animals regarding this patent application were approved by the Ethics Committee on Animal Use of Oswaldo Cruz Foundation (CEUA License - LW-23/10).

In this study, Wistar rats of both sexes were used, weighing between 200 and 250 g, coming from the Laboratory Animal Breeding Center of Fundação Oswaldo Cruz. As previously described (Coelho *et al*., 2008), the animals were sacrificed by exposure to air atmosphere enriched with CO₂. Then, the anterior cervical region was opened so that the trachea could be located and removed. It was then transferred to a Petri dish containing Krebs solution with the following composition (mM): NaCl (118), KC1 (4.8), CaCl₂ (2.5), MgSO₄ (1.2), KH₂PO₄ (1.2), NaHCO₃ (24), and glucose (11). The total segment was divided into fragments of about 3 to 4 rings, which were kept in another Petri dish containing Krebs solution. Each fragment was mounted vertically on a 10 ml cuvette with Krebs solution maintained at 37 °C and aerated with carbogen mixture (95% O₂ and 5% CO₂). The lower rod is fixed to the cuvette base and the top portion was attached to the isometric transducer for measuring the voltage variation of the fragment. The transducer was connected to a device which transforms the voltage variation in digital record. The fragments were subjected to a basal voltage of 1 g and were calibrated, so that the subsequent contractions could be expressed as a percentage of this 1 g voltage. The solutions were introduced inside the cuvettes with the aid of an automatic pipette. The end of the tip was always placed at the same height and position, without touching the muscle. The tracheal rings were initially contracted with 2.5 µM of carbachol. When the contractions reached the plateau, each segment was washed until the total relaxation of the smooth muscle. The compounds mexiletine (30-1000 µM) JME-173 (30-100 µM) and JME-207 (10-100 µM) were added 10 minutes before the addition of increasing concentrations of carbachol (10⁻⁸-10⁻⁴ M). All results were expressed as percentage of the contraction produced by 2.5 µM of carbachol (Coelho et *al.,* 2008).

### B. Statistical Analysis

The values of the mean ± Standard Error of the Mean of the groups investigated were statistically analyzed using the test of analysis of variance (ANOVA), followed by Student-Newman-Keuls test. *p*-Values lower than or equal to 0.05 were considered significant.

### C. Results

Table 2 shows that the compounds JME-173 and JME-207 were eguieffective in blocking the contractile response of rat tracheal rings induced by the muscarinic agonist carbachol (10 µM), with IC₅₀ values of 44.4 and 40.9 µM, respectively. Pre-treatment for 10 minutes with JME-173 and JME-207 (100 µM) reached levels of inhibition of the muscarinic contraction of the order of 98% and 93%, respectively. The analogues were about 10 times more potent than the prototype mexiletine, which under the same conditions inhibited 50% of the response (IC₅₀) at the concentration of 466.4 µM, reaching the blocking of about 88% of the muscarinic contraction at the concentration of 1000 µM.

**Table 2: Potency values (IC₅₀) and maximum effect (EMAX) of inhibition of the response of (10 µM) carbachol-induced contraction on rat tracheal rings pretreated for 10 minutes with mexiletine, JME-173 or JME-207, representatives of the class of aryloxyalkylamines. Data represent the mean ± SEM from 4 to 7 tracheal rings.**

| **Compounds** | **IC₅₀ (µM)** | **EMAX** |
|---|---|---|
| Mexiletine | 466.4 | 87.7 ± 4.9 |
| JME-173 | 44.3 | 97.9 ± 4.7 |
| JME-207 | 40.9 | 92.9 ± 4.3 |

### Example 5 - Effect of mexiletine, JME-173 and JME-207 in the relaxation of trachea pre-contracted by carbachol.

### A. Method and Evaluation

To assess the potential relaxing effect of the respiratory smooth muscle, rat tracheas were obtained and maintained in an isolated organ bath, as previously described (Coelho et al., 2008). The tracheal segments were then pre-contracted with carbachol at the concentration of 2.5 µM and subjected to increasing concentrations of the tested compounds. All results were expressed as percentage of the contraction produced by carbachol (Coelho et al., 2008).

### B. Statistical Analysis

The values of the mean ± Standard Error of the Mean of the groups investigated were statistically analyzed using the test of analysis of variance (ANOVA), followed by Student-Newman-Keuls test. *p*-Values lower than or equal to 0.05 were considered significant.

### C. Results

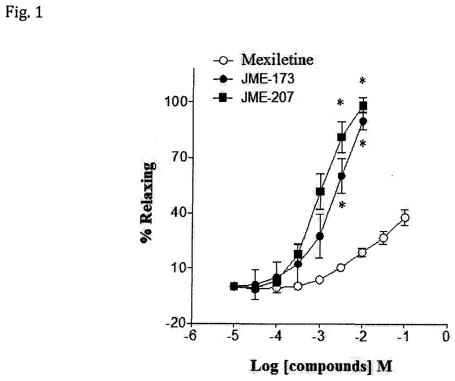

Figure 1 shows the relaxing effect of the compounds JME-173 and JME-207 in comparison to the prototype compound mexiletine. It was observed that at conditions of pre-contraction by carbachol, the addition of mexiletine (10 µM - 100 mM), in isolated tracheal ring system, caused a relaxation which increased with increasing concentration of the prototype mexiletine, to achieve a maximum relaxation effect of 40% ± 3% (n = 10). On the other hand, treatments with JME-173 and JME-207 (10 µM - 10 mM) resulted in maximum relaxation of 118% ± 21% (N=11) (mean ± SEM) and 111% ± 8% (N=9), respectively. Under these conditions, the EC₅₀ values of relaxation for mexiletine, JME-173 and JME-207 were 146.4 mM ± 39.2 mM (mean ± SEM; N=10) , 3.1 mM ± 0.8 mM (N=9) and 1.0 mM ± 0.3 mM (N=8), respectively. The findings show that the relaxing potency of the analogues is significantly higher than that evidenced by the prototype in this preparation. More specifically, the compounds JME-173 and JME-207 were, in this order, 47 and 146 times more potent as relaxation inducers than mexiletine. The results reinforce the interpretation that these analogues have therapeutic application in the control of spasm of airways.

### Example 6 - Antispasmodic activity of the compound JME-173 evaluated in the system of anaphylactic contraction of tracheal ring.

### A. Method and Evaluation

In this study, Wistar rats of both sexes were used, weighing between 200 and 250 g, coming from the Laboratory Animal Breeding Center of Fundação Oswaldo Cruz. As described previously (da Costa et al., 2007), the animals were sensitized by injection into the dorsal subcutaneous tissue with mixture containing 50 µg of ovalbumin and 5 mg of aluminum hydroxide on days 0 and 7. On the 14^{th} day after the first sensitization, the animals were sacrificed for the removal of the trachea. After a stabilization period of 30 minutes, the tracheal rings were contracted initially with carbachol (2.5 µM) for testing the feasibility and reproducibility of the responses of the preparation.

### Treatment and anaphylaxis challenge

The tracheal rings were exposed to increasing concentrations of JME-173 (3-30 µM), or carrier (0.9% NaCl) for 30 minutes before the triggering of the contractile response triggered by ovalbumin (100 µg/ml). Salmeterol (30 µM) was used as reference treatment. The responses were expressed as mean ± Standard Error of the Mean of at least 5 tracheal segments. All results were expressed as percentage of the contraction produced by 2.5 µM of carbachol.

### B. Statistical Analysis

The values of the mean ± Standard Error of the Mean of the groups investigated were statistically analyzed using the test of analysis of variance (ANOVA), followed by Student-Newman-Keuls test. The statistical evaluation of the data obtained for treatment with salmeterol was performed using the Student's T-test. *p*-Values lower than or equal to 0.05 were considered significant.

### C. Results

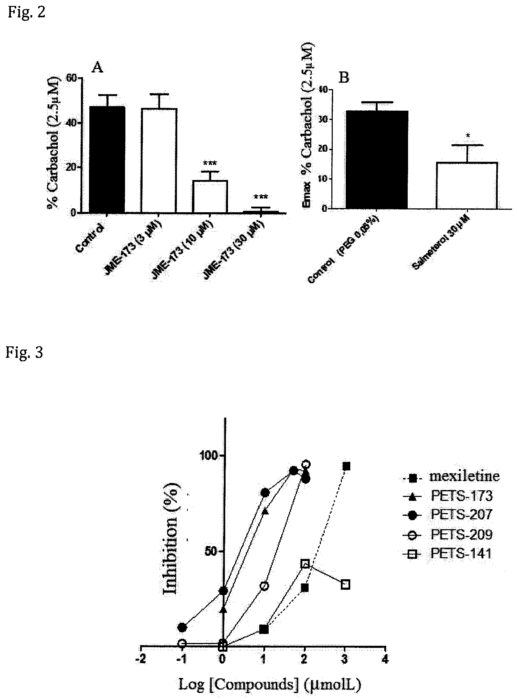

Figure 2A shows the antispasmodic effect, concentration-dependent, of the compound JME-173 on the contractile response induced by the addition of the allergen. It was observed that JME-173 inhibited 50% of the anaphylactic contraction response (EC₅₀) at the concentration of 8.3 µM. The response was completely inhibited after treatment with 30 µM of JME-173. Importantly, at the same concentration (30 µM), the blocking exhibited by salmeterol was only 52% (Figure 2B). The findings demonstrate that JME-173 was more potent than salmeterol in blocking anaphylactic contraction. It was also evident the greater antispasmodic activity of JME-173 in the anaphylactic contraction system, in comparison to the blocking exhibited by this compound on carbachol-induced contraction.

### Example 7 - Anti-anaphylactic activity of the compounds mexiletine, JME-173, JME-207 and JME-209, evaluated in the system of degranulation of sensitized mast cells induced by antigen.

### A. Method and Evaluation

For this study, mast cells of the RBL-2H3 line were used, as previously reported (Beaven et al., 1987). The cells were maintained in D-MEM medium supplemented with 15% fetal bovine serum, penicillin (100 IU/ml) and streptomycin (0.1 mg/ml), and placed in an oven at 37 °C and atmosphere of 5% CO₂ until reaching confluence. The cells were then dissociated from the plate using trypsin, centrifuged at 1000 rpm for 5 minutes and distributed in 48-well plates at a density of 125,000 cells per well. The cells were sensitized with monoclonal DNP-specific IgM (1 µg/mL) diluted in the same medium used for the cultivation and maintained in the oven for 20 hours. After this period, the cells were washed with Tyrode-gelatin and subjected to treatment with increasing concentrations of JME-173, JME-207, JME-209 or mexiletine for 60 minutes. Then, incubation was performed with DNP-BSA (10 ng/ml) for a further period of 60 minutes. After this period, 10 µL of supernatant were collected from each well and added to a 96-well plate. The cells were lysed with 200 µL of 0.1% Triton X-100 and 10 µL of the lysate of each plate were added to the 96-well plate. Then, 40 µL of substrate for the β-hexosaminidase enzyme were added to the samples. After 40 minutes of reaction, reaction stopping solution (0.2 M glycine) was added, generating a colorimetric response, which was measured by spectrophotometer (λ = 405 nm).

The compounds JME-173, JME-207, JME-209 and mexiletine were also evaluated for their cytotoxic potential, based on the Alamar Blue assay, as previously reported (Czekanska, 2011). In this test, the compound terfenadine was used as positive control.

### B. Statistical Analysis

The results were expressed as inhibition percentage. Statistical differences were determined by using tests of analysis of variance, followed by the Student-Newman-Keuls test. p-Values lower than or equal to 0.05 were considered significant.

### C. Results

Several local anesthetic agents, such as lidocaine, inhibit mast cell degranulation induced by mechanisms mediated or not mediated by IgE, by blocking calcium channels (Yanagi *et al.*, 1996). Our results showed that the compound mexiletine also inhibited the anaphylactic degranulation of mast cells at concentrations ranging from 100 µM to 1000 µM (Figure 3). The same figure shows that the analogues studied JME-173, JME-207 and JME-209 were equally effective in blocking mast cell degranulation caused by exposure to the allergen, evidencing, however, greater potency of the analogues studied when compared to mexiletine.

Table 3 shows the comparative potency values (IC₅₀) and efficacy (EMAX) of the compounds studied. All of them inhibited by about 100% the degranulation response, whereas IC₅₀ values decreased from 381.8 µM, obtained after treatment with mexiletine, to 28.6 µM, 3.4 µM and 2.3 µM after JME-209, JME-173 and JME-207, respectively.

These results, obtained with mast cells passively sensitized with IgE, indicate that the analogues JME-173, JME-207 and JME-209 were capable of inhibiting the anaphylactic activation of mast cells with higher potency (up to two orders of magnitude) when compared to the prototype.

**Table 3 - Comparative values of inhibition potency (IC50) and maximum effect (EMAX) of mexiletine, JME-207, JME-173 and JME-209, of the classes of diphenyloxyalkylamines and aryloxyalkylamines, relative to the blocking of anaphylactic mast cell degranulation.**

| Compounds | IC₅₀ (µM) | EMAX |
|---|---|---|
| Mexiletine | 381.8 | 100 |
| JME-207 | 2.3 | 94 |
| JME-173 | 3.4 | 97 |
| JME-209 | 28.6 | 100 |

### Exemple 8 - Bronchodilator activity of the compounds JME-207 and JME-209 in vivo

### A. Method and Evaluation

A/J mice of both sexes were used, weighing between 18 and 20 g, coming from the Laboratory Animal Breeding Center of Oswaldo Cruz Foundation. Using barometric whole-body plethysmography (Buxco Research System, Wilmington, NC), bronchospasm responses caused by subsequent inhalations of methacholine (12, 25 and 50 mg/ml for 2.5 minutes, 5-minute intervals) were measured in standard A/J mice, awake, not immobilized, as previously reported (Coelho *et al*., 2008; Hamelmann *et al.,* 1997). Penh measures in response to methacholine challenge were performed 1 hour and 3 hours after treatment with JME-207 and JME-209 (30 and 100 mg/kg) administered orally (gavage).

### B. Statistical Analysis

The results were expressed as mean ± Standard Error of the Mean. Statistical differences were determined by using tests of analysis of variance, followed by the Student-Newman-Keuls test. *p*-Values lower than or equal to 0.05 were considered significant.

### C. Results

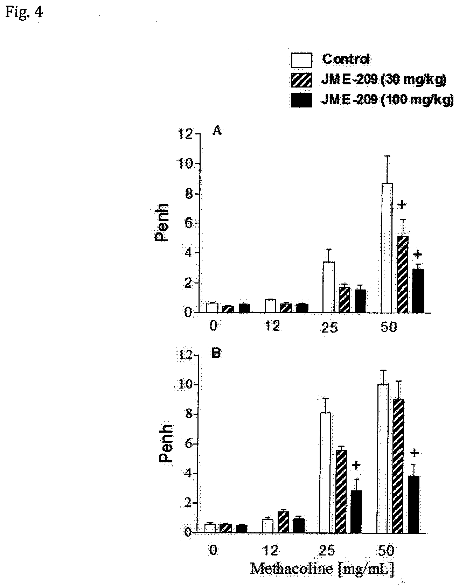

Figure 4 shows the effect of treatment with JME-209 (30 and 100 mg/kg, orally) or carrier (0.9% NaCl) on the response of increase of Penh (indicative of increase in lung resistance) induced by methacholine challenge (12-50 mg/ml) in the times 1 hour and 3 hours after treatment. There was a slight blocking of cholinergic bronchospasm with both doses used (30 and 100 mg/kg) in the analysis conducted 1 hour after treatment. The blocking shown to be active only at the highest dose, when the tests of stimulation response with methacholine was repeated 3 hours after treatment, suggesting that the compound has an action time of at least 3 hours when the substance is administered orally at a dose of 100 mg/kg.

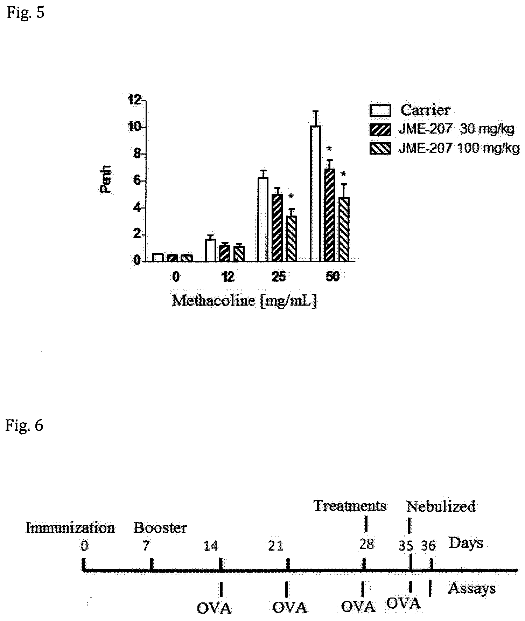

Similar results were obtained when the animals were treated with JME-207. Administered orally, at doses of 30 and 100 mg/kg, the compound significantly inhibited the response of methacholine-induced bronchoconstriction 3 hours after treatment (Figure 5). The results together demonstrate the antispasmodic activity of the compounds JME-209 and JME-207, *in vivo,* confirming our data obtained in isolated organ system (*in vitro*)*.*

### Example 9 - Therapeutic effect of the compounds JME-141, JME-173, JME-207 and JME-188 on lung inflammation and hyperreactivity in asthma model in mice.

### A. Method and Evaluation

Male A/J mice (18-20 g), coming from the Laboratory Animal Breeding Center of Oswaldo Cruz Foundation, were used in the experiments. The sensitizing and antigen challenge procedures used in this study followed the experimental protocol shown in Figure 6. The animals were previously sensitized with a mixture of ovalbumin (OVA) (50 µg) (Grade V; Sigma, St. Louis, MO, USA) and aluminum hydroxide (5 mg) administered subcutaneously on day 0 with boost on day 14 (equal suspension administered intraperitoneally). Nasal instillations of OVA (25 µg/25 µl in sterile 0.9% NaCl) were administered on days 14, 21, 28 and 35, with the hyperresponsiveness analysis performed 24 hours after the last challenge. As shown in Figure 6, the treatments with JME-141, JME-173, JME-207, and JME-188 were carried out only on days 28 and 35, 1 hour before the challenge with OVA, by nebulization for 30 minutes. That is, the treatments took place after the installation of asthma framework, reflecting a therapeutic action of the respective compounds analyzed.

The effect of the treatments on bronchial hyperreactivity was investigated by measuring the resistance changes and pulmonary elastance, using invasive barometric plethysmography whole-body system (Buxco, USA), as previously described (Olsen et al., 2012).

### B. Statistical Analysis

The results were expressed as mean ± Standard Error of the Mean. Statistical differences were determined by using tests of analysis of variance, followed by the Student-Newman-Keuls test. *p*-Values lower than or equal to 0.05 were considered significant.

### C. Results

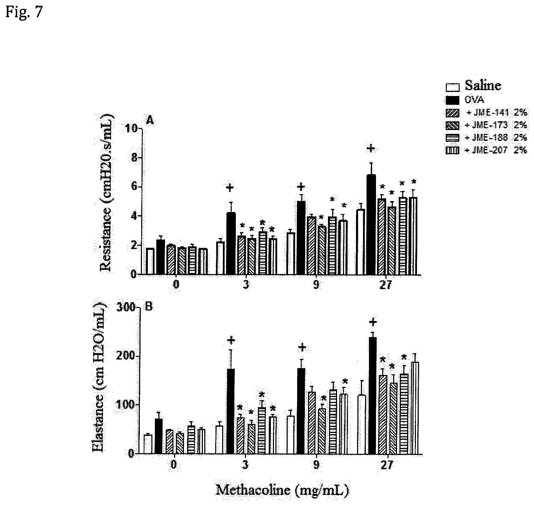

As shown in Figure 7, the nebulization of the animals for 30 minutes with the compounds JME-141, JME-173, JME-207 and JME-188 (2%), starting from the third week of allergen challenge, abolished the framework of hyperreactivity of airways, observed in animals antigenically sensitized and challenged, treated only with carrier (Tween-80, 0.2%). The inhibition was found to be effective both in lung resistance increasing response, as in the increase of lung elastance observed after exposure of methacholine.

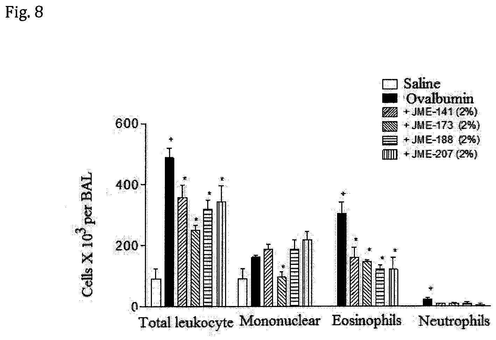

All compounds were equally active in blocking leukocyte infiltration, evaluated by bronchoalveolar lavage, especially for the inhibition of eosinophilic infiltration, inhibited by 50% by all compounds tested (Figure 8). Analyses of cellularity were also carried out 24 hours after the last antigen challenge.

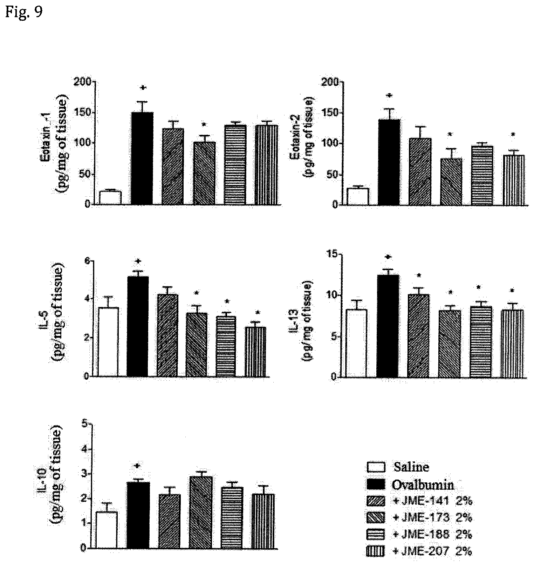

Our results indicated that the inhibition of hyperresponsiveness and cellular recruitment responses, as evidenced by treatment with JME-173, was shown to be associated to the blocking of pro-inflammatory cytokine generation including eotaxin-2, IL-5 and IL-13, without change in the increased levels of the anti-inflammatory cytokine IL-10 (Figure 9).

The production of cytokines IL-5 and IL-13 was equally sensitive to the treatment with JME-207 or JME-188, but only the latter inhibited eotaxin-2, while both failed to modify the increased production of eotaxin-1. These data indicate that, with minor particularities, the compounds JME-141, JME-173, JME-207 and JME-188, administered via nebulization (2%), are active in blocking lung inflammation and hyperreactivity associated with the asthmatic response. The joint results also suggest that the inhibition of the generation of the pro-inflammatory Th₂ cytokines can be involved in the blocking of pathological features of asthma profile observed in this model.

### Example 10 - Effect of nebulization with JME-173 on inflammation, mucus production and lung remodeling in murine model of asthma

### A. Method and Evaluation

Male A/J mice (18-20 g), coming from the Laboratory Animal Breeding Center of Oswaldo Cruz Foundation, were used in the experiments. The sensitization procedures, antigen challenge and treatment used in this study followed the experimental protocol shown in Figure 6. Histological techniques were used for the quantification of mucus and peribronchial fibrosis, following previously reported and validated experimental protocols (Serra *et al*., 2012).

### B. Statistical Analysis

The results were expressed as mean ± Standard Error of the Mean. Statistical differences were determined by using tests of analysis of variance, followed by the Student-Newman-Keuls test. *p*-Values lower than or equal to 0.05 were considered significant.

### C. Results

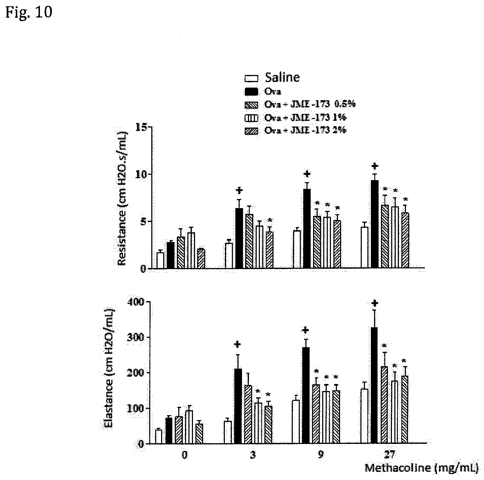

The treatment by nebulization with JME-173 in the concentrations of 0.5%, 1% or 2% for 30 minutes, started at the third week of allergen challenge (Figure 10), confirmed the antiasthmatic effect of this compound.

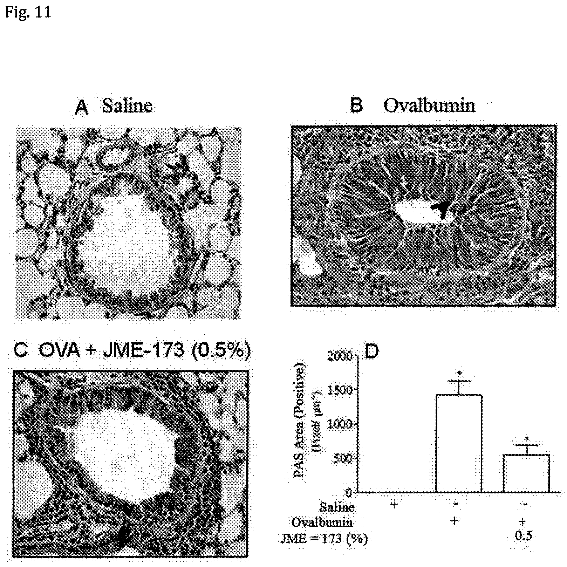

It was evident in the three aerosol concentrations tested that the compound JME-173 was able to block the response of airway hyperreactivity even at the lowest concentration (0.5%), as illustrated in Figure 11. In this model, challenge with ovalbumin caused a significant increase in the amount of mucus present in the airways of the sensitized animals (micrograph B of figure 11) (PAS staining, arrowheads), when compared with control animals challenged with 0.9% saline (micrography B). It is noted in Figure 11, part C, that the exacerbated mucus production observed in asthmatic mice was substantially inhibited by the treatment with JME-173 (0.5%). Figure 11D shows the result of quantitative analysis, where it is evident that JME-173 inhibited mucus production by about 70%.

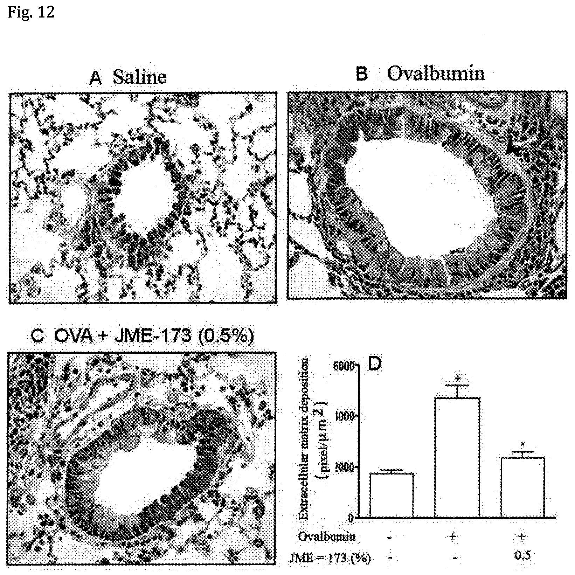

The staining of histological sections of lung tissue with Gömöri trichrome stain evidenced a marked accumulation of extracellular matrix in the peribronchial region (indicated by the arrowhead) in the animals challenged with ovalbumin (Figure 12, part B), when compared to animals in the negative control group (Part A). The treatment with JME-173 clearly abolished the fibrotic response, as illustrated by the representative image, as well as by the quantitative analysis conducted based on the morphometry (Figure 12 Part C and D, respectively). Taken together, the data suggest that the nebulization treatment with JME-173 is capable of reversing airways hyperreactivity, as well as inhibits mucus production in the lower airways and peribronchial fibrosis caused by intranasal instillation of allergen agent in sensitized mice.

### Example 11 - Effect of the compound JME-209 on lung inflammation and airways hyperreactivity caused by LPS

### A. Method and Evaluation

Male A/J mice (18-20 g), coming from the Laboratory Animal Breeding Center of Oswaldo Cruz Foundation, were used in the experiments. The mice were anesthetized with halothane aerosol (Cristália, SP, Brazil) to receive intranasal administration of LPS (25 µg/25 µl 0.9% NaCl, instillation) or 0.9% NaCl (25 µl) (negative control). The animals were pretreated with JME-209 (30 and 100 mg/kg, orally) 1 hour before instillation of LPS, and analysis of the impact of treatment on leukocyte recruitment in the airspace was performed 18 hour after challenge. Obtaining of bronchoalveolar lavage, as well as the total and differential leukocyte counts carried out in this effluent, were made as previously described (Kummerle et al., 2012). Thus, after 18 hrs of LPS instillation, the mice were sacrificed by terminal anesthesia with thiopental (500 mg/kg). Then, they had the trachea dissected and cannulated. Bronchoalveolar lavage (BAL) was performed by 3 consecutive lavages of 800 µl of PBS containing EDTA (10 mM). The lavages were then subjected to centrifugation (1500 rpm - 10 minutes) and the cell "pellet" resuspended in the volume of 0.5 ml of PBS/EDTA solution 10 mM. The total leukocyte count from the lavage was performed in a Neubauer chamber by light microscopy (100x magnification), diluting an aliquot of the cell suspension from the lavage in Turk liquid (1:40). The differential counting was performed on cytocentrifuged, which were stained with May-Grunwald-Giemsa and assessed using oil immersion objective (1000x magnification)(Kummerle *et al*., 2012).

The airway hyperreactivity was also assessed 18 hours after LPS, by exposing the animals to increasing concentrations of aerosolized methacholine (3-27 mg/ml) in FinePoint R/C Buxco® system (Buxco Electronics, Sharon, CT, USA). The mice were anesthetized with Nembutal (60 mg/kg, i.p.) for the tracheostomy procedure and connection of the animal to mechanical ventilation and pneumotachograph of the FinePoint platform. The neuromuscular activity was blocked with pancuronium bromide (1 mg/kg, i.v.) to enable the pulmonary resistance records (cm H20/mL/s) and elastance (cm H20/mL) in each respiratory cycle, as previously reported (Olsen *et al*., 2011).

### B. Statistical Analysis

The results were expressed as mean ± Standard Error of the Mean. Statistical differences were determined by using tests of analysis of variance, followed by the Student-Newman-Keuls test. *p*-Values lower than or equal to 0.05 were considered significant.

### C. Results

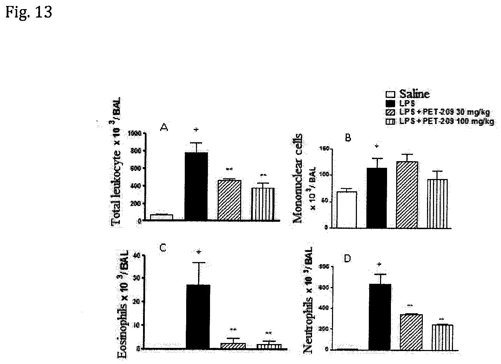

In this model of acute pulmonary inflammation caused by endotoxin, the treatments with JME-209 (30 and 100 mg/kg, orally) administered 1 hour before LPS (25 µg/animal), inhibited leukocyte infiltration in bronchoalveolar space, in particular reducing the levels of eosinophils and neutrophils, without significantly altering the increase in the number of mononuclear cells (Figure 13) .

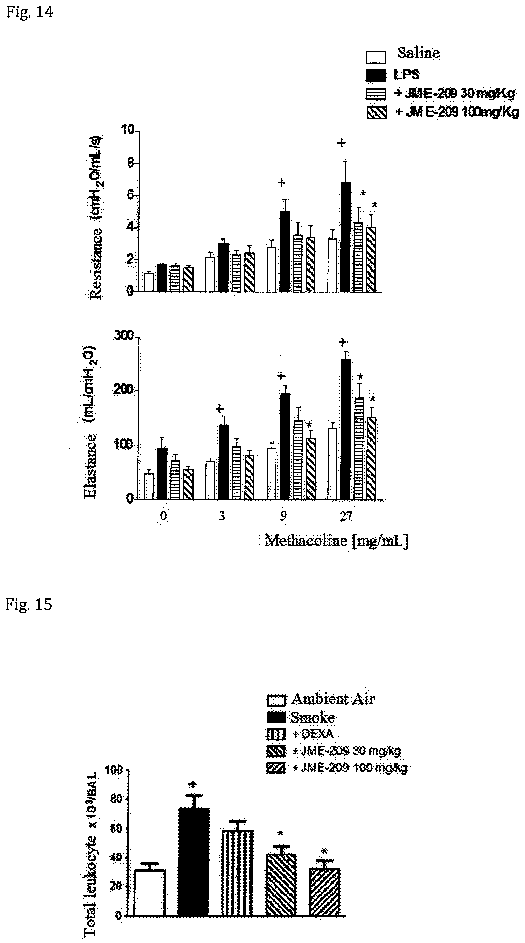

Under these conditions, the treatment also inhibited the mechanical ventilation changes (airway hyperreactivity), represented by the significant increase in lung resistance and elastance values, which are indicators of air flow reduction in the central airways and reduction of expansion capacity of the lung parenchyma, respectively (Figure 14)

In conclusion, the results show that the pulmonary inflammation and airway hyperreactivity caused by LPS were clearly inhibited by the oral treatment with JME-209, suggesting that this compound has potential for inhibiting chronic pulmonary inflammatory diseases, such as asthma and chronic obstructive pulmonary disease (COPD).

### Example 12 - Protective effect of JME-209 on acute airway inflammation induced by tobacco smoke in mice

### A. Method and Evaluation

Male A/J mice (18-20 g), coming from the Laboratory Animal Breeding Center of Oswaldo Cruz Foundation, were used in the experiments. The animals were placed in a chamber and subjected to an atmosphere enriched with 100 ml of smoke from 4 filter cigarettes (trade mark) for 1 minute on four consecutive days. Control animals were exposed to the condition in which cigarette smoke was replaced by equal volume of ambient air (Castro *et al*., 2009).

The treatments with dexamethasone (1 mg/kg) or JME-209 (30 and 100 mg/kg) were carried out orally 1 hour before each exposure to smoke. The compounds were dissolved in 0.9% NaCl just prior to administration.

Obtaining of bronchoalveolar lavage, as well as the total and differential leukocyte counts carried out in this effluent, were made as previously described (Olsen *et al.,* 2011). Thus, after 24 hrs of the last exposure to cigarette smoke, the mice were sacrificed by terminal anesthesia with thiopental (500 mg/kg). Then, they had the trachea dissected and cannulated BAL was performed by 3 consecutive lavages of 800 µl of PBS containing EDTA (10 mM). The lavages were then subjected to centrifugation (1500 rpm - 10 minutes) and the cell "pellet" resuspended in the volume of 0.5 ml of PBS/EDTA solution 10 mM. The total leukocyte count from the lavage was performed in a Neubauer chamber by light microscopy (100x magnification), diluting an aliquot of the cell suspension from the lavage in Turk liquid (1:40). The differential counting was performed on cytocentrifuged, which were stained with May-Grunwald-Giemsa and assessed using oil immersion objective (1000x magnification).

### B. Statistical Analysis

The results were expressed as mean ± Standard Error of the Mean. Statistical differences were determined by using tests of analysis of variance, followed by the Student-Newman-Keuls test. *p*-Values lower than or equal to 0.05 were considered significant.

### C. Results

In this model of acute pulmonary inflammation by cigarette smoke established in mice (Castro *et al.,* 2009), the treatment with JME-209 (30 and 100 mg/kg, orally) 1 hr prior to challenge with smoke significantly inhibited the accumulation of leukocytes in the bronchoalveolar space, while the treatment with dexamethasone (3 mg/kg, orally) was ineffective (Figure 15).

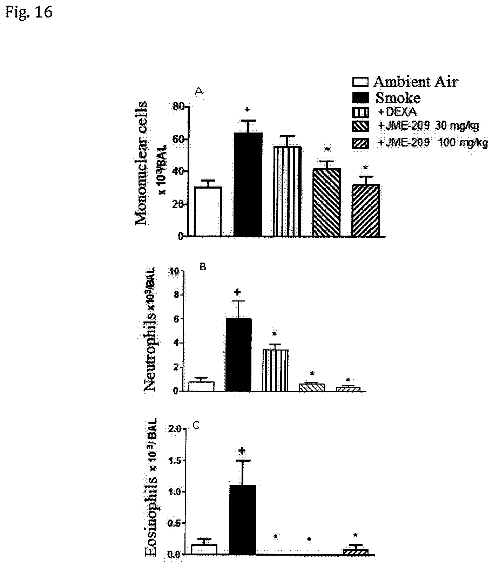

The increase in total leukocytes resulted substantially from increases in the numbers of neutrophils, eosinophils and mononuclear cells in the bronchoalveolar effluent, which changes were blocked by JME-209. The treatment with the steroidal anti-inflammatory Dexamethasone (1 mg/kg, orally) also inhibited the slight increase in the number of eosinophils, but was unable to inhibit the accumulation of mononuclear cells and only partially inhibited neutrophil infiltration (Figure 16).

In conclusion, considering that cigarette smoke is a major cause of asthma and COPD worsening, the results presented herein strongly suggest that the treatment with JME-209 has the potential to prevent pulmonary inflammation associated with cigarette smoke, an important pathogenesis factor in these patients.

The derivatives of the present invention, as described herein, are usually administered as a pharmaceutical composition. Such compositions may be prepared by procedures well known in the pharmaceutical art and comprise at least one active compound of the invention.

The compounds of this invention are usually administered in a pharmaceutically effective amount. The actual amount of compound administered will be typically determined by a physician, in the light of the relevant circumstances, including the condition to be treated, the chosen route of administration, the compound administered, the age, weight and response of the individual patient, the severity of the symptoms of the patient, and so forth.

The derivatives and compositions described in this patent application can be administered to a subject, preferably a mammal, more preferably a human, to treat and/or prevent the disease by any suitable route.

The compositions containing the derivatives of the present invention may be formulated as:
(1) tablets, capsules, powders for reconstitution;
(2) oral solution;
(3) oral suspension; or
(4) solution for inhalation.

The compositions of the present invention are typically formulated with suitable carriers and may be exemplified as follows.

### (1) Based formulation for tablets, capsules and powders for reconstitution

| Component (function) | Pharmaceutically acceptable carriers | Amount (%) |
|---|---|---|
| Active ingredient | | 10.0 - 80.0 |
| Disintegrant | Croscarmellose sodium, sodium starch glycolate, crospovidone | 1.0 - 7.0 |
| Glidant | Colloidal silicon dioxide, talc | 0.5 - 4.0 |
| Binder | Polyvinylpyrrolidone (PVP), hydroxypropyl methylcellulose (HPMC), hydroxypropyl cellulose (HPC) | 0.5 - 4.0 |
| Diluent | Lactose, mannitol, calcium phosphate, microcrystalline cellulose, pregelatinized starch | 5.0 - 70.0 |
| Flavoring | Strawberry, cherry, orange | 0.05 - 1.00 |
| Colorant | FD&C red no. 3, FD&C yellow no. 6 | qs |
| Sweetener | Aspartame, sodium cyclamate, sucralose, saccharin | 0.05 - 0.5 |
| Lubricant | Magnesium stearate, calcium stearate, stearic acid, sodium stearyl fumarate | 0.5 - 3.0 |

### (2) Oral solution

| Component (function) | Pharmaceutically acceptable carriers | Amount (%) |
|---|---|---|
| Active ingredient | | 10.0 - 80.0 |
| Antioxidant | Ascorbic acid, potassium metabisulfite, sodium metabisulfite, Butylated hydroxyanisole (BHA), Butylated hydroxytoluene (BHT), citric acid | 0.005 - 2.0 |
| Preservatives | Sodium benzoate, potassium benzoate, propylparaben, methylparaben, butylparaben, potassium sorbate | 0.05 - 0.5 |
| pH corrector | Citric acid, fumaric acid, triethanolamine | |
| Flavoring | Strawberry, cherry, orange | 0.05 - 1.00 |
| Colorant | FD&C red no. 3, FD&C yellow no. 6 | 0.01 - 0.5 |
| Sweetener | Aspartame, sodium cyclamate, sucralose, saccharin, sucrose | 0.05 - 60.0 |
| Solubilizer | Propylene glycol, cyclodextrin | qs |
| Solvent | Water | qs |

### (3) Oral suspension

| Component (function) | Pharmaceutically acceptable carriers | Amount (%) |
|---|---|---|
| Active ingredient | | 10.0 - 80.0 |
| Suspending agent | Xanthan gum, sodium carboxymethyl cellulose, methylcellulose | 0.5 - 5.0 |
| Antioxidant | Ascorbic acid, potassium metabisulfite, sodium metabisulfite, Butylated hydroxyanisole (BHA), Butylated hydroxytoluene (BHT), citric acid | 0.005 - 2.0 |
| Preservatives | Sodium benzoate, potassium benzoate, propylparaben, methylparaben, butylparaben, potassium sorbate | 0.05 - 0.5 |
| pH corrector | Citric acid, fumaric acid, triethanolamine | |
| Sweetener | Aspartame, sodium cyclamate, sucralose, saccharin, sucrose | 0.05 - 60.0 |
| Flavoring | Strawberry, cherry, orange | 0.05 - 1.00 |
| Colorant | FD&C red no. 3, FD&C yellow no. 6 | qs |
| Solvent | Water | qs |

### (4) Solution for inhalation

| Component (function) | Pharmaceutically acceptable carriers | Amount (%) |
|---|---|---|
| Active ingredient | | 1.0 - 20.0 |
| Isotonizing agent | Sodium chloride | 1.0 -10.0 |
| Surfactant | Oleic acid, lecithin, Span 85, PVP K25 | 0.5 - 5.0 |
| pH corrector | Sulfuric acid | qs |
| Solvent | Water | qs |

The carriers (components) described above for the compositions are merely representative. Other materials, as well as processing techniques and the like, are set in specific literature, such as Remington's Pharmaceutical Sciences, 18th edition, 1990, Mack Publishing Company, Easton, Pennsylvania, 18042.

Although the present invention has been described with respect to specific embodiments, it is evident that many alternatives and variations are apparent to those skilled in the art. These alternatives and variations should be considered to be supported by the scope of the claims.

Documents belonging to the state of the art of the knowledge of the inventors and cited in the present descriptive report are listed below.
1. **Patente** US 3.659.019
2. **Patente** US 3.954.872
3. **Patente** US 4.031.244
4. Beaven MA, Maeyama K, Wolde-Mussie E, Lo TN, Ali H, Cunha-Melo JR (1987). Mechanism of signal transduction in mast cells and basophils: studies with RBL-2H3 cells. Agents Actions 20(3-4): 137-145.
5. Brightling CE, Gupta S, Gonem S, Siddiqui S (2012). Lung damage and airway remodelling in severe asthma. Clin. Exp. Allergy 42(5): 638-649.
6. Brody H (2012). Chronic obstructive pulmonary disease. Nature 489(7417): S1.
7. Campbell RW (1987). Mexiletine. N. Engl. J. Med. 316(1): 29-34.
8. Castro P, Nasser H, Abrahao A, Dos Reis LC, Rica I, Valenca SS, et al. (2009). Aspirin and indomethacin reduce lung inflammation of mice exposed to cigarette smoke. Biochem. Pharmacol. 77(6): 1029-1039.
9. Coelho LP, Serra MF, Pires AL, Cordeiro RS, Rodrigues e Silva PM, dos Santos MH, et al. (2008). 7-Epiclusianone, a tetraprenylated benzophenone, relaxes airway smooth muscle through activation of the nitric oxide-cGMP pathway. J. Pharmacol. Exp. Ther. 327(1): 206-214.
10. Czekanska EM (2011). Assessment of cell proliferation with resazurin-based fluorescent dye. Methods Mol. Biol. 740: 27-32.
11. da Costa JC, Olsen PC, de Azeredo Siqueira R, de Frias Carvalho V, Serra MF, Alves LA, et al. (2007). JMF2-1, a lidocaine derivative acting on airways spasm and lung allergic inflammation in rats. J. Allergy Clin. Immunol. 119(1): 219-225.
12. Dance A (2012). Health impact: Breathless. Nature 489 (7417) : S2-3.
13. Groeben H, Foster WM, Brown RH (1996). Intravenous lidocaine and oral mexiletine block reflex bronchoconstriction in asthmatic subjects. Am. J. Respir. Crit. Care Med. 154(4 Pt 1): 885-888.
14. Hamelmann E, Schwarze J, Takeda K, Oshiba A, Larsen GL, Irvin CG, et al. (1997). Noninvasive measurement of airway responsiveness in allergic mice using barometric plethysmography. Am. J. Respir. Crit. Care Med. 156(3 Pt 1): 766-775.
15. Jarvis B, Coukell AJ (1998). Mexiletine. A review of its therapeutic use in painful diabetic neuropathy. Drugs 56(4): 691-707.
16. Kummerle AE, Schmitt M, Cardozo SV, Lugnier C, Villa P, Lopes AB, et al. (2012). Design, Synthesis, and Pharmacological Evaluation of N-Acylhydrazones and Novel Conformationally Constrained Compounds as Selective and Potent Orally Active Phosphodiesterase-4 Inhibitors. J. Med. Chem.
17. Marmura MJ, Passero FC, Jr., Young WB (2008). Mexiletine for refractory chronic daily headache: a report of nine cases. Headache 48(10): 1506-1510.
18. Monk JP, Brogden RN (1990). Mexiletine. A review of its pharmacodynamic and pharmacokinetic properties, and therapeutic use in the treatment of arrhythmias. Drugs 40(3): 374-411.
19. Neher E, Sakmann B (1992). The patch clamp technique. Sci. Am. 266(3): 44-51.
20. Olsen PC, Coelho LP, da Costa JC, Cordeiro RS, Silva PM, Martins MA (2012). Two for one: Cyclic AMP mediates the anti-inflammatory and anti-spasmodic properties of the non-anesthetic lidocaine analog JMF2-1. Eur. J. Pharmacol. 680: 102-107.
21. Olsen PC, Ferreira TP, Serra MF, Farias-Filho FA, Fonseca BP, Viola JP, et al. (2011). Lidocaine-derivative JMF2-1 prevents ovalbumin-induced airway inflammation by regulating the function and survival of T cells. Clin. Exp. Allergy 41(2): 250-259.
22. Serra MF, Anjos-Valotta EA, Olsen PC, Couto GC, Jurgilas PB, Cotias AC, et al. (2012). Nebulized lidocaine prevents airway inflammation, peribronchial fibrosis, and mucus production in a murine model of asthma. Anesthesiology 117(3): 580-591.
23. Yanagi H, Sankawa H, Saito H, Iikura Y (1996). Effect of lidocaine on histamine release and Ca2+ mobilization from mast cells and basophils. Acta Anaesthesiol. Scand. 40(9): 1138-1144.
24. Remington's Pharmaceutical Sciences, 18th edição, 1990, Mack Publishing Company, Easton, Pennsylvania, 18042.

## Claims

1. Diphenyloxyalkylamine derivatives and aryloxyalkylamine derivatives selected from the structures below:
| **Structure** | **Code** |
|---|---|
| 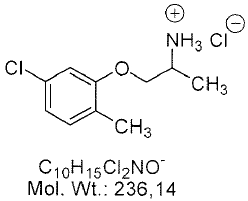 | JME-170 |
| 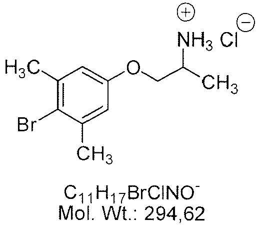 | JME-173 |
| 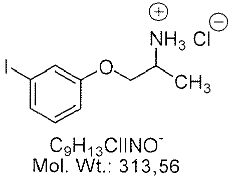 | JME-141 |
| 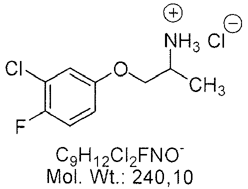 | JME-188 |
| 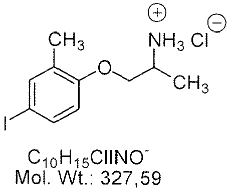 | JME-207 |
| 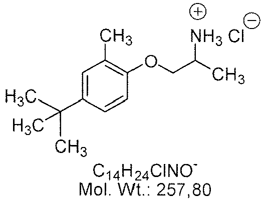 | JME-208 |
| 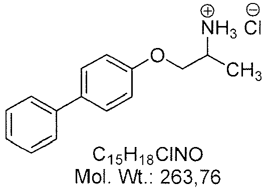 | JME-209 |
| 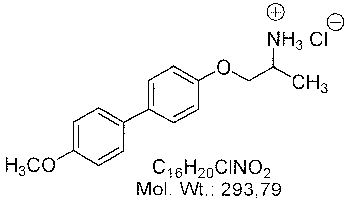 | JME-209-1 |
| 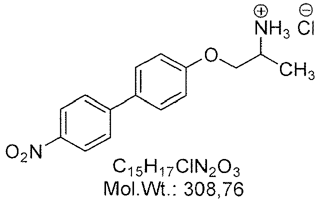 | JME-209-2 |
| 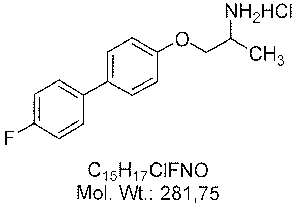 | JME-209-3 |
| 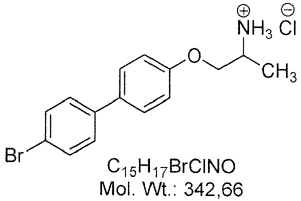 | JME-209-4 |
| 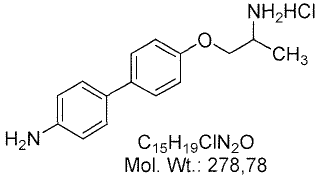 | JME-209-5 |
| 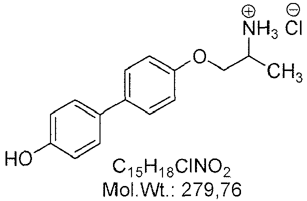 | JME-209-6 |
| 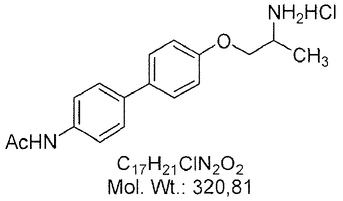 | JME-209-7 |
| 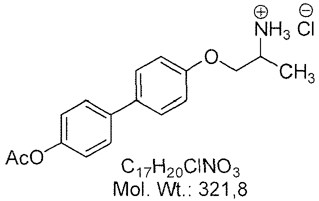 | JME-209-8 |

2. A pharmaceutical composition **characterized by** containing at least one of the derivatives as defined in claim 1, or one of its salts formed by organic or mineral acids, and a pharmaceutically acceptable carrier.

3. A pharmaceutical composition according to claim 2 formulated as tablets, capsules, powders for reconstitution; oral solution; oral suspension; or inhalation solution.

4. A pharmaceutical composition according to claim 3 **characterized in that** the composition in the form of tablets, capsules and powders for reconstitution contains agents selected from the group consisting of disintegrants; glidants; binders, diluents, flavorings, colorants, sweeteners or lubricants.

5. A pharmaceutical composition according to claim 3 **characterized in that** the composition in the form of oral solution contains agents selected from the group consisting of antioxidants, preservatives, pH correctors, flavorings, colorants, sweeteners, solubilizers or solvents.

6. A pharmaceutical composition according to claim 3 **characterized in that** the composition in the form of oral suspension contains agents selected from the group consisting of suspenders, antioxidants, preservatives, pH correctors, sweeteners, flavorings, colorants or solvents.

7. A pharmaceutical composition according to claim 3 **characterized in that** the composition in the form of inhalation solution contains agents selected from the group consisting of isotonizing, surfactants, pH correctors or solvents.

8. Composition as defined in any one of claims 2 to 7 for use in the treatment, prevention or inhibition of pulmonary inflammatory disease by administration in a pharmacologically effective amount through any route of administration.

## Patentansprüche

1. Diphenyloxyalkylaminderivate und Aryloxyalkylaminderivate, ausgewählt aus den nachfolgenden Strukturen:
| **Struktur** | **Code** |
|---|---|
| 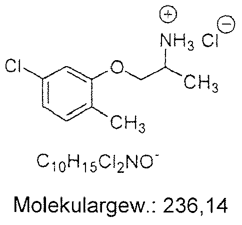 | JME-170 |
| 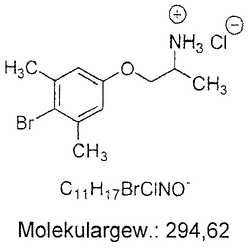 | JME-173 |
| 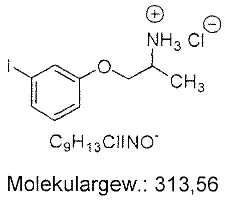 | JME-141 |
| 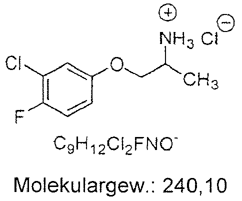 | JME-188 |
| 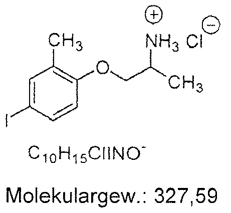 | JME-207 |
| 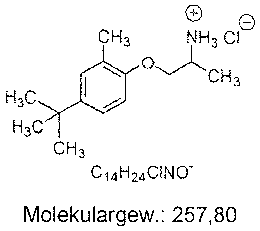 | JME-208 |
| 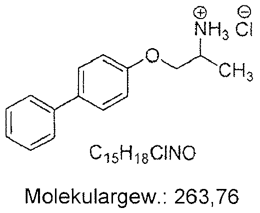 | JME-209 |
| 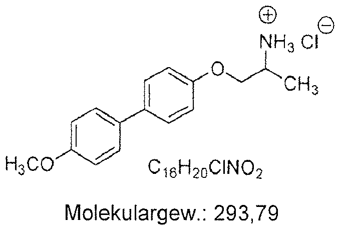 | JME-209-1 |
| 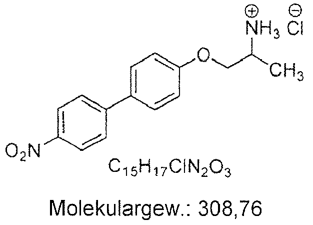 | JME-209-2 |
| 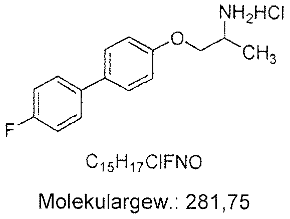 | JME-209-3 |
| 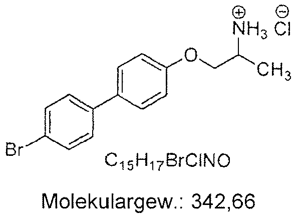 | JME-209-4 |
| 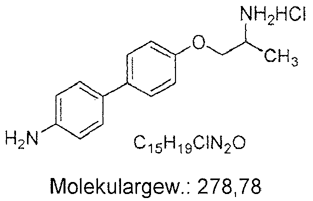 | JME-209-5 |
| 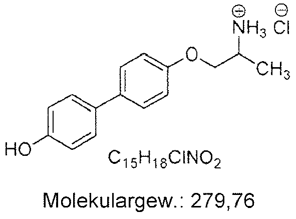 | JME-209-6 |
| 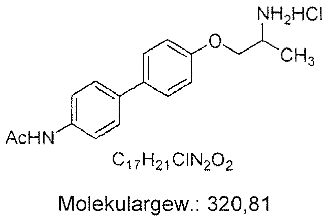 | JME-209-7 |
| 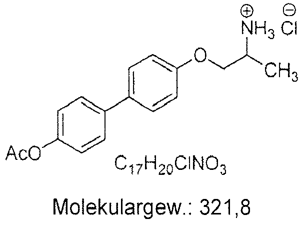 | JME-209-8 |

2. Pharmazeutische Zusammensetzung, **gekennzeichnet durch** das Enthalten von mindestens einem der in Anspruch 1 definierten Derivate oder einem seiner Salze, gebildet durch organische oder mineralische Säuren, und einem pharmazeutisch annehmbaren Träger.

3. Pharmazeutische Zusammensetzung nach Anspruch 2, formuliert als Tabletten, Kapseln, Pulver zur Rekonstitution; orale Lösung; orale Suspension oder Inhalationslösung.

4. Pharmazeutische Zusammensetzung nach Anspruch 3, **dadurch gekennzeichnet, dass** die Zusammensetzung in der Form von Tabletten, Kapseln und Pulvern zur Rekonstitution Mittel enthält, ausgewählt aus der Gruppe, bestehend aus Sprengmitteln; Gleitmitteln; Bindemitteln; Verdünnungsmitteln, Geschmacksstoffen, Farbstoffen, Süßungsmitteln oder Schmierstoffen.

5. Pharmazeutische Zusammensetzung nach Anspruch 3, **dadurch gekennzeichnet, dass** die Zusammensetzung in der Form von oralen Lösungen Mittel enthält, ausgewählt aus der Gruppe, bestehend aus Antioxidantien, Konservierungsmitteln, pH-Wert-Korrekturmitteln, Geschmacksstoffen, Farbstoffen, Süßungsmitteln, Solubilisierungsmitteln oder Lösungsmitteln.

6. Pharmazeutische Zusammensetzung nach Anspruch 3, **dadurch gekennzeichnet, dass** die Zusammensetzung in der Form von oralen Suspensionen Mittel enthält, ausgewählt aus der Gruppe, bestehend aus Suspensionsmitteln, Antioxidantien, Konservierungsmitteln, pH-Wert-Korrekturmitteln, Süßungsmitteln, Geschmacksstoffen, Farbstoffen oder Lösungsmitteln.

7. Pharmazeutische Zusammensetzung nach Anspruch 3, **dadurch gekennzeichnet, dass** die Zusammensetzung in der Form von Inhalationslösungen Mittel enthält, ausgewählt aus der Gruppe, bestehend aus Isotonisierungsmitteln, Surfactants, pH-Wert-Korrekturmitteln oder Lösungsmitteln.

8. Zusammensetzung wie in einem der Ansprüche 2 bis 7 definiert, zur Verwendung bei der Behandlung, Prävention oder Inhibierung von pulmonaren entzündlichen Erkrankungen durch Verabreichung in einer pharmakologisch wirksamen Menge über irgendeinen Verabreichungsweg.

## Revendications

1. Dérivés de diphényloxyalkylamine et dérivés d'aryloxyalkylamine choisis parmi les structures ci-dessous :
| **Structure** | **Code** |
|---|---|
| 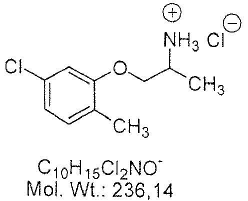 | JME-170 |
| 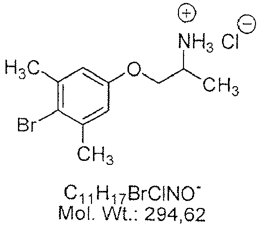 | JME-173 |
| 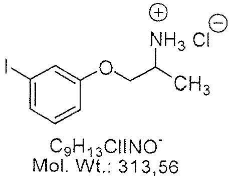 | JME-141 |
| 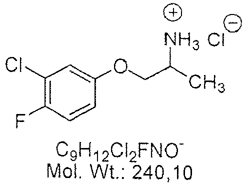 | JME-188 |
| 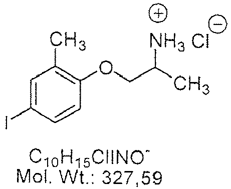 | JME-207 |
| 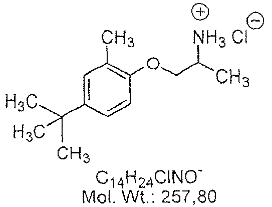 | JME-208 |
| 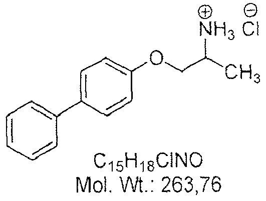 | JME-209 |
| 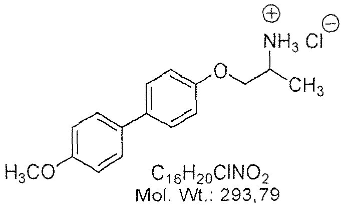 | JME-209-1 |
| 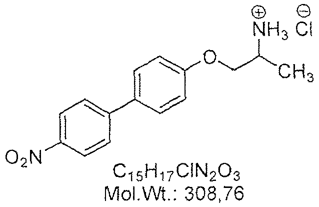 | JME-209-2 |
| 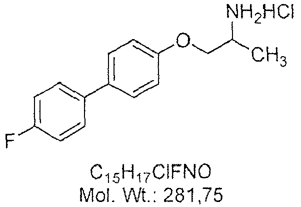 | JME-209-3 |
| 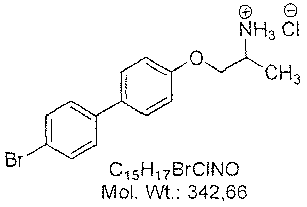 | JME-209-4 |
| 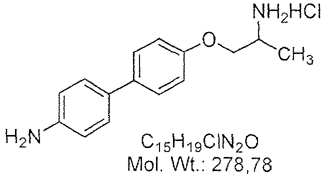 | JME-209-5 |
| 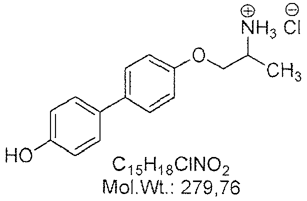 | JME-209-6 |
| 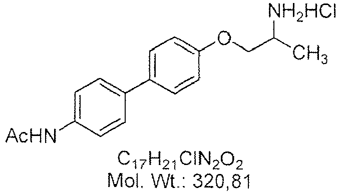 | JME-209-7 |
| 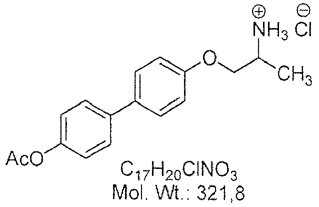 | JME-209-8 |

2. Composition pharmaceutique **caractérisée en ce qu'**elle contient au moins l'un des dérivés tels que définis dans la revendication 1, ou l'un de ses sels formés par des acides organiques ou minéraux, et un véhicule pharmaceutiquement acceptable.

3. Composition pharmaceutique selon la revendication 2 formulée sous la forme de comprimés, capsules, poudres pour reconstitution ; solutions à usage oral ; suspensions à usage oral ; ou solutions pour inhalation.

4. Composition pharmaceutique selon la revendication 3, **caractérisée en ce que** la composition sous la forme de comprimés, capsules et poudres pour reconstitution contient des agents choisis dans le groupe constitué par les délitants ; les agents de glissement ; les liants, les diluants, les aromatisants, les colorants, les édulcorants et les lubrifiants.

5. Composition pharmaceutique selon la revendication 3, **caractérisée en ce que** la composition sous la forme d'une solution à usage oral contient des agents choisis dans le groupe constitué par les antioxydants, les conservateurs, les correcteurs de pH, les aromatisants, les colorants, les édulcorants, les solubilisants et les solvants.

6. Composition pharmaceutique selon la revendication 3, **caractérisée en ce que** la composition sous la forme d'une suspension à usage oral contient des agents choisis dans le groupe constitué par les agents de mise en suspension, les antioxydants, les conservateurs, les correcteurs de pH, les édulcorants, les aromatisants, les colorants et les solvants.

7. Composition pharmaceutique selon la revendication 3, **caractérisée en ce que** la composition sous la forme d'une solution pour inhalation contient des agents choisis dans le groupe constitué par les agents d'isotonicité, les tensioactifs, les correcteurs de pH et les solvants.

8. Composition selon l'une quelconque des revendication 2 à 7 pour une utilisation dans le traitement, la prévention ou l'inhibition d'une maladie inflammatoire pulmonaire par administration en une quantité efficace du point de vue pharmacologique par n'importe quelle voie d'administration.
